(19) **Europäisches Patentamt**
**European Patent Office**
**Office européen des brevets**

(11) **EP 2 125 931 B1**

(12) **EUROPEAN PATENT SPECIFICATION**

(45) Date of publication and mention
of the grant of the patent:
**09.03.2011 Bulletin 2011/10**

(51) Int Cl.:
**C08G 63/682** (2006.01)  **C08G 63/91** (2006.01)

(21) Application number: **08724538.7**

(22) Date of filing: **16.01.2008**

(86) International application number:
**PCT/US2008/000558**

(87) International publication number:
**WO 2008/097416 (14.08.2008 Gazette 2008/33)**

(54) **VINYL ENDS MEASURING METHOD**

VERFAHREN ZUR MESSUNG VON VINYLENDEN

PROCÉDÉ DE MESURE D'EXTRÉMITÉS VINYLE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **02.02.2007 US 701599**

(43) Date of publication of application:
**02.12.2009 Bulletin 2009/49**

(73) Proprietor: **EASTMAN CHEMICAL COMPANY
Kingsport TN 37660 (US)**

(72) Inventor: **SPAUGH, Arthur, Thaler, Jr.
Kingsport, TN 37660 (US)**

(74) Representative: **Best, Michael et al
Lederer & Keller
Patentanwälte
Prinzregentenstrasse 16
80538 München (DE)**

(56) References cited:
- **TORU AMARI, KUMIKO NISHIMURA, KIMIKO MINOU, AKIRA KAWABATA, YUKIHIRO OZAKI: "End-group characterization of homo- and copolyesters of cyclohexane-1,4-dimethanol" JOURNAL OF POLYMER SCIENCE: PART A: POLYMER CHEMISTRY, vol. 39, 2001, pages 665-674, XP002480035**
- **MA Y., AGARWAL U.S., VEKEMANS J.A.J.M., SIKKEMA D.J.: "NMR based determination of minute acid functionality: end-groups in PET" POLYMER, vol. 44, 2003, pages 4429-4434, XP002480049**

**Description**

1. Field of the Invention:

The invention relates to a method for the measurement of vinyl ends concentration in a polyester polymer. More particularly, the invention relates to a method for the measurement of vinyl ends in a polyester polymer using a liquid solution comprising at least one fluorinated carboxylic acid or fluorinated carboxylic anhydride to form a mixture, optionally heating the liquid solution or the mixture to a temperature between about 30 °C and 300 °C. subjecting said mixture to an analysis wherein a quantitative signal for a fluorine atom or a fluorine-containing compound is produced; and calculating the quantity or concentration of vinyl ends from said quantitative signals.

2. Background of the Invention

Polyester polymers and especially polyethylene terephthalate polymer are widely used for various applications such as sheets, boards, extrusion blow molded bottles, extruded laminates, containers, and beverage bottles. Certain physical characteristics make polyester polymers and polyester polymer particles such as polyethylene terephthalate (PET) desirable for packaging applications include impact strength, moldability, clarity, transparency, and color. However, depending upon the specific application, there are other characteristics and properties that are desirable especially for extrusion blow molded articles.

For example, polyester polymers and especially PET (polyethylene terephthalate) is used extensively in water bottle and carbonated soft drink ("CSD") bottle applications. A typical method by which these bottles are produced is by melt processing of PET pellets to form bottle preforms followed by extrusion blow molding to form bottles. In this application, concentration levels of acetaldehyde (AA) are a concern. Of specific concern are two types of acetaldehyde (AA). The first is residual or free AA contained in polyester pellets or polyester particles used as raw material in extrusion blow molding. A second type of AA is preform AA or the AA generated when PET pellets are melt processed to make bottle preforms. AA precursors in the solid polyester particles, chemical compounds or chemical functional groups which may react upon degradation and/or upon melting of the polyester can produce unacceptable levels of AA in the preforms. In addition, new AA precursors are formed when the polyester polymer is held in the molten state, as in the case of an injection molding process to make bottle preforms. Acetaldehyde has a noticeable taste and can be highly undesirable in beverage container applications. When performs are blown into bottles, unacceptably high AA levels are those that adversely impact the taste of the beverage contained in these bottles. Relatively tasteless beverages such as water are particularly negatively impacted by the taste of AA. Many water bottle applications require lower levels of preform AA than carbonated soft drink ("CSD") bottle applications.

Another example of a normally desirable characteristic of the polyester polymer melts and any subsequent polyester particles produced by solidification of the melt is that of low vinyl ends concentration. Vinyl ends as represented by the formula: $-CO_2-CH=CH_2$ are known AA precursors. One commonly accepted mechanism by which AA is generated in molten polyester is the internal chain scission of a polyester polymer chain to form a vinyl end group and a carboxylic acid end group. The vinyl end group can react with a hydroxyethyl end group or water to form residual or free AA and a new internal ester linkage. There is a common perception that a high concentration of vinyl ends is thus undesirable due to the ability of the vinyl end to react to form AA during subsequent melt processing of the polyester polymer.

Because subsequent production of AA can be problematic, there are many advantages in having an accurate quantitative measurement of the concentration of AA precursors, and specifically a quantitative measurement of the concentration of vinyl ends. For example, by having a quantitative measurement of vinyl ends, it may be advantageous for bottle producers to adjust the operating conditions of extrusion blow molding equipment (e.g. operating temperature, residence time *inter alia)* to minimize the amount of AA generated during the molding process. In another example, by having a quantitative measurement of vinyl ends, it may be advantageous for PET pellet producers to adjust the operating conditions of known solid state polymerization processes to remove sufficient AA to make the polyester polymer product acceptable for water bottle and/or CSD applications. In still another example, by having a quantitative measurement of vinyl ends, it may be advantageous to adjust the amount of AA scavenger added to a polyester polymer to match the amount of AA generated anticipated from decomposition of vinyl ends.

Other than AA generation, there are other advantages of having a quantitative measurement of vinyl ends concentration in polyester polymers. For example, a smaller number of olefin terminals (e.g. vinyl ends) is usually preferred for improving melt heat stability of the modified and unmodified polyester polymers. In particular, it is usually undesirable to have large variations in intrinsic viscosity between pellets, preforms, and/or bottles since this could lead to inconsistency in the end application. For beverage applications such as carbonated soft drink or water bottles there is usually is not more than 0.04 dUg, preferably not more than 0.03 dUg, and most preferably not more than 0.02 dUg difference in intrinsic viscosity. Additionally, it is known that vinyl ends may also polymerize to polyvinyl esters which may be responsible for yellow coloration of PET.

There are known methods for measuring olefin terminal ends concentration in highly modified polyester polymers are reported such as reported in J. Polymer Science A, volume 39, issue 5, 665-674 (reference A). However, the methods described are generally associated with the measurement of cyclovinylidene and methyl cyclohexene end groups rather than vinyl ends. The cyclovinylidene and methyl cyclohexene end groups are characteristic of cyclohexanedimethanol-modified polyethylene terephthalate polymers. Reference A does refer to two methods of measuring vinyl ends concentration. Both methods described have significant shortcomings. The first method utilizes taking a proton 1H-NMR (proton nuclear magnetic resonance) spectra. The second method is a modified method based on a journal article (J. G. M. Aalbers, and G. D. B. van Houwelingen, Fresenius Z Anal. Chem., 314(5),472-475(1983)) wherein vinyl ends are reacted with bromine and followed by measurement of coulometric bromination. This method requires a second measurement to correct for other compounds which react with bromine. The method is not specific for the analysis of the vinyl end group and even with the correction described in the method; the results will be confounded by other reactions which consume bromine.

As reported in Reference A, the amount of vinyl ends in PET homopolymer is small compared with the content of cyclovinylidene and methyl cyclohexene end groups in cyclohexanedimethanol-modified polymers. Further Reference A also indicate that quantitative differences in the values of vinyl ends concentration using both methods for PET homopolymer may be either a result of experimental error or a result of the presence of other unsaturated species other than vinyl ends.

These methods are not entirely satisfactory because they have poor sensititivity toward detecting vinyl ends at low concentration levels and one method is not specific toward the detection of vinyl ends only. Hence, there is a need to develop a method for measurement of vinyl ends concentration in polyester polymers and particularly polyethylene terephthalate polymer that is specific toward measurement of vinyl ends, that is sensitive and accurate to low concentration levels of vinyl ends, and that can be effectively utilized when measuring vinyl ends in PET homopolymers.

### 3. Summary of the Invention

An objective of this invention is to provide a method for the measurement of vinyl ends concentration in a polyester polymer. In an embodiment of this invention, a method for the measurement of vinyl ends concentration in a polyester polymer is provided comprising:

a). dissolving said polyester polymer with a liquid solution comprising at least one fluorinated carboxylic acid or fluorinated carboxylic anhydride to form an anhydrous mixture;
b). subjecting said mixture to an analysis wherein a quantitative signal for fluorine or a fluorine-containing compound is produced; and
c). calculating the quantity or concentration of vinyl ends from said quantitative signals.

In another embodiment of this invention, a method for the measurement of vinyl ends concentration in a polyester polymer is provided comprising:

a). dissolving said polyester polymer with a liquid solution comprising at least one fluorinated carboxylic acid or fluorinated carboxylic anhydride to form an anhydrous mixture;
b). optionally heating said liquid solution or said mixture to a temperature between about 30 °C and 300 °C;
c). subjecting said mixture to an analysis wherein a quantitative signal for fluorine or a fluorine-containing compound is produced; and
d). calculating the quantity or concentration of vinyl ends from said quantitative signals.

### 4. Detailed Description of the Invention

The present invention may be understood more readily by reference to the following detailed description of the invention.

In the method of this invention, a "polyester polymer" comprises a dicarboxylic acid moiety comprising one or more dicarboxylic acids or their ester forming derivatives and a diol moiety. Examples of dicarboxylic acid moieties include, but are not limited to, terephthalic acid, derivates of terephthalic acid, isophthalic acid, derivates of isophthalic acid, naphthalene-2,6-dicarboxylic acid, derivatives of naphthalene-2,6-dicarboxylic acid, or mixtures thereof. Other examples of dicarboxylic acid moieties include, aromatic dicarboxylic acids having 8 to 14 carbon atoms, aliphatic dicarboxylic acids having 4 to 12 carbon atoms, or cycloaliphatic dicarboxylic acids having 8 to 12 carbon atoms.

Examples of diol moieties include, but are not limited to, ethylene glycol, cycloaliphatic diols preferably having 6 to 20 carbon atoms and/or aliphatic diols preferably having 3 to 20 carbon atoms. More specific examples of such diols include diethylene glycol; triethylene glycol; 1,4-cyclohexanedimethanol; propane-1,3-diol; butane-1,4-diol; pentane-1,5-diol; hexane-1,6-diol; 3-methylpentanediol- (2,4); 2-methylpentanediol-(1,4); 2,2,4-trimethylpentane-diol-(1,3); 2,5- ethylhexanediol-(1,3); 2,2-diethyl propane-diol-(1, 3); hexanediol-(1,3); 1,4-di-(hydroxyethoxy)-benzene; 2,2-bis-(4-hydroxycyclohexyl)-propane; 2,4- dihydroxy-1,1,3,3-tetramethyl-cyclobutane; 2,2-bis-(3-hydroxyethoxyphenyl)-propane; and 2,2-bis-(4-hydroxypropoxyphenyl)-propane, and diethylene glycol.

Of particular interest are polyester polymers containing repeating alkylene aryl units, such as alkylene terephthalate or alkylene naphthalate repeat units in the polymer chain. More specific examples of these repeating units include ethylene terephthalate, ethylene naphthalate, and trimethylene terephthalate. An example of a polyester polymer is one which comprises:

(i) a carboxylic acid component comprising at least 80 mole% of the residues of terephthalic acid, derivates of terephthalic acid, naphthalene-2,6-dicarboxylic acid, derivatives of naphthalene-2,6-dicarboxylic acid, or mixtures thereof, and
(ii) a hydroxyl component comprising at least 80 mole% of the residues of ethylene glycol or propane diol,
based on 100 mole percent of carboxylic acid component residues and 100 mole percent of hydroxyl component residues in the polyester polymer.

All compounds containing carboxylic acid group(s) or derivative(s) thereof that are part of the polyester polymer comprise the "carboxylic acid component residue." The mole % of all the compounds containing carboxylic acid group (s) or derivative(s) thereof which are in the polyester polymer sum to 100.

All compounds containing hydroxyl group(s) or derivatives thereof that are part of the polyester polymer comprise the hydroxyl component. The mole % of all the compounds containing hydroxyl group(s) or derivatives thereof which are in the polyester polymer sum to 100.

Another example of a polyester polymer is one which comprises:

(a) a carboxylic acid component comprising at least 90 mole%, or at least 92 mole%, or at least 96 mole% of the residues of terephthalic acid, derivates of terephthalic acid, naphthalene-2,6-dicarboxylic acid, derivatives of naphthalene-2,6-dicarboxylic acid, or mixtures thereof, more preferably terephthalic acid or derivates of terephthalic acid, and
(b) a hydroxyl component comprising at least 90 mole%, or at least 92 mole%, or at least 96 mole % of the residues of ethylene glycol or propane diol, more preferably ethylene glycol,

based on 100 mole percent of the carboxylic acid component residues and 100 mole percent of the hydroxyl component residues in the polyester polymer.

In an embodiment of this invention, a method for the measurement of vinyl ends concentration in a polyester polymer is provided.

A polyester polymer is combined with a liquid solution comprising at least one fluorinated carboxylic acid or fluorinated carboxylic anhydride, preferably at least one perfluorinated carboxylic acid or perfluorinated carboxylic anhydride. Fluorinated carboxylic acids generally constitute linear or branched aliphatic or aromatic carboxylic acids, wherein at least one of the carbon-bonded hydrogen atoms has been replaced by fluorine. As it is used herein, the term perfluorinated means that all of the carbon-bonded hydrogen atoms have been replaced by fluorine. Examples of suitable fluorinated acids and anhydrides include, but are not limited to, monofluoroacetic acid, difluoroacetic acid, trifluoroacetic acid, pentafluoropropionic acid, heptafluorobutyric acid, perfluoropentanoic acid, perfluorohexanoic acid, chlorodifluoroacetic acid, 2,2,3,3-tetrafluoropropionic acid, 3,3,3-trifluoropropionic acid, alpha,alpha,alpha-trifluorotoluic acid, pentafluorobenzoic acid, and anhydrides thereof.

The amount of polyester polymer utilized is not limited. However, one advantage of the method of this invention is that sensitivity and accuracy of the method is such that only a small amount of polyester polymer is required to obtain quantitative measurement of vinyl ends concentration. Typically, the amount of polyester polymer combined with liquid solution ranges from at least 0.01 grams, or at least 0.05 grams, or at least 0.1 grams, or at least 0.3 grams, and up to about 1.0 grams, or up to about 5.0 grams, or up to about 10 grams, or up to about 100 grams. The amount of liquid solution is generally an amount required to dissolve the polyester polymer at ambient temperatures. The amount of fluorinated or perfluorinated acid or anhydride is generally an amount necessary to provide a sufficient number of fluorinated or perfluorinated carboxylic acid moieties (derivates from the acid or anhydride) to react with the number of vinyl ends in the polyester.

Perfluorinated acids and anhydrides, especially trifluoroacetic acid and trifluoroacetic anhydride and mixtures thereof, are particularly effective in the method of this invention due to their ability to solubilize or dissolve a broad number of polyester polymers. In the specific example of PET (polyethylene terephthalate) polymer and even more specifically PET polymer pellets with a high degree of crystallinity normally used for CSD and water bottle applications, it is difficult to find appropriate solvents in which to dissolve these pellets.

In U.S. 5,852,164, there is described a method for counting vinyl ends of a highly modified polyester polymer in a chloroform solvent using a 1H-NMR (proton nuclear magnetic resonance) which is similar or possibly the same as that described in reference A. Highly modified polyester polymers, for example polyethylene terephthalate modified with greater than 30 mole percent cyclohexanedimethanol are generally amorphous as opposed to crystalline. In the instance where in the measurement of vinyl ends for PET polymer pellets with a high degree of crystallinity and large average molecular weight, the methods described in U.S. 5,852,164 and reference A are generally not applicable since the polyester would either not dissolve in the chloroform solvent, or there would be a need to utilize excessive solvent to polyester weight ratios such that the sensitivity and/or accuracy of subsequent counting of vinyl ends would be impacted. Further the other methods described do not produce measurements of vinyl ends concentrations with the sensitivity or the specificity of the present invention. In particular, in discussing the utilized methods therein, Reference A indicates that a shortcoming of the coulometric bromination method is a lack of specificity toward vinyl ends. Further, in the discussion section of the Reference A, it is further speculated that the difference between concentration values obtained via the 1H-NMR method and the modified coulometric bromination method may be a result of either experimental error or a result of the presence of other unsaturated species in the polymer. Additionally, Reference A indicates that the shortcoming of the 1H-NMR method is a lack of sensitivity, as the vinyl end group could only be detected in PET samples subjected to significant thermal degradation. Presumably, thermal degradation of the polymer was necessary to increase the number of vinyl ends so that the 1H-NMR method could be used to obtain a quantitative measurement of vinyl ends.

In this invention, perfluorinated acids and anhydrides, especially trifluoroacetic acid and trifluoroacetic anhydride and mixtures thereof, are particularly useful since they create a chemical structure within the polymer that is sensitive to fluorine nuclear magnetic resonance (FMR) measurement and which produce a distinct FMR signal. Hence, unlike other methods, the method of this invention is both specific and sensitive to the counting of vinyl ends in polyester polymers. In particular, the sensitivity of the method is such that vinyl ends are detectable in a range from at least 0.01 millimoles per kilogram of polymer (mmol/kg), or at least 0.05 mmol/kg, or at least 0.1 mmol/kg, and up to about 0.5 mmol/kg, or up to about 10.0 mmol/kg, or up to about 100 mmol/kg, based on the mass of a polyester sample of about 0.5 grams. Compared with other methods, this invention is more sensitive and specific for measurement of the vinyl end group. The reported range of the measurement of the bromination method is 1 to 20 mmol/kg. The reported precision of the bromination method is 0.25 mmol/kg for vinyl ends concentration between 1 to 20 mmol/kg. The 1H-NMR method does not report a precision value or range of measurement.

Because of the high solubility of polyester polymers in perfluorinated acids and anhydrides, dissolving of the polyester polymer into the liquid solution can proceed unaided. However, in an embodiment of this invention, it is provided that the combining of polyester polymer with liquid solution can be subjected to agitation and/or subjected to heating to hasten dissolution of the polyester polymer and reaction of the vinyl end groups. Agitation can be accomplished by any means known in the art. For example, agitation can be accomplished by mechanical stirring. Further, heating can be accomplished by any means known in the art. In a preferred embodiment, the liquid solution or the mixture of liquid solution and polymer may be heated up to about 30°C, or up to about 50°C, or up to about 150°C, or up to about 300°C. In the instance where the liquid solution comprises trifluoroacetic acid and trifluoroacetic anhydride under anhydrous conditions, heating and agitation may be used to hasten reaction of the vinyl end groups with excess perfluorinated moieties.

The liquid solution and/or mixture of liquid solution with polymer may also comprise other chemical compounds. For example, the liquid solution may be a mixture of trifluoroacetic acid, trifluoroacetic anhydride, and a solvent. Particularly suitable solvents include liquids in which polyester polymers exhibit some solubility (chloroform, for example). In general, suitable solvents may include aliphatic and aromatic hydrocarbons, esters, and ethers. These solvents may be halogenated derivatives of aliphatic hydrocarbons, esters, or ethers. Further, any hydrogen atoms that comprise the molecules of these solvents may be substituted with or replaced with deuterium or tritium. The liquid solution and/or mixture of liquid solution with polymer may also comprise other chemical compounds comprising fluorine, in as far as the presence of those fluorine-containing compounds do not interfere with the measurement of vinyl ends concentration. Examples of suitable compounds include, but are not limited to, butane, pentane, hexane, heptane, octane, nonane, decane, undecane, dodecane, cyclopentane, cyclohexane, cycloheptane, 2,2,4-trimethyl pentane, acetonitrile, butyl acetate, tert-butyl methyl ether, 1,4-dioxane, ethyl ether, isopropyl ether, butyl ether, 2-methoxyethyl ether, tetrahydrofuran, nitromethane, benzene, toluene, xylene, ethylbenzene, nitrobenzene, benzonitrile, anisole, pyridine, chlorobenzene, bromobenzene, dichlorobenzene, trichlorobenzene, dibromobenzene, methylene chloride, methylene chloride-d2, chloroform, chloroform-d, chlorobutane, carbon tetrachloride dichloromethane, dichloroethane, dichloroethylene, trichloroethane, trichloroethylene, tetrachloroethane, tetrachloroethane-d2, tetrachloroethylene chlorodifluoroacetic acid. Additionally, the presence of water or alcohols in the liquid solution and/or mixture of liquid solution with polymer is discouraged. Fluorinated and perfluorinated acid moieties react with vinyl ends on the polymer. The presence of water or alcohols in the liquid may lead to undesirable chemical reactions. For example, the presence of water may lead to the formation of hydrolysis products. Under some circumstances, for example when using a liquid solution and/or mixture of liquid solution with polymer comprising a perfluorinated carboxylic acid anhydride such as trifluoroacetic anhydride, it may be preferable to utilize a stoichiometric excess of anhydride. However, a large excess of anhydride is generally not desirable. For example, when a liquid solution comprising trifluoroacetic acid and trifluoroacetic anhydride is utilized, subsequent analysis of quantitative FMR signals for specific fluorine compounds indicative of vinyl ends may be indistinguishable FMR signals associated with trifluoracetic anhydride.

In the instance where an excess amount of fluorinated or prefluorinated acid moieties are utilized, there is provided embodiments of the present invention wherein the excess amount of fluorinated or perfluorinated acid moieties are either removed or reacted to a compound or compounds that do not interfere in the quantitative measurement of vinyl ends. For example, when a liquid solution and/or mixture of liquid solution with polymer comprises trifluoroacetic acid and trifluoroacetic anhydride, it may be advantageous to reduce the concentration of trifluoroacetic anhydride moieties prior to subjecting the mixture to an analysis wherein a quantitative signal for fluorine is produced. If the mixture is subjected to FMR (fluorine nuclear magnetic resonance) measurement, the trifluoroacetic anhydride can cause problems with measurements due to its large signal size in the FMR spectrum compared with the vinyl ends group signal. However, it also may be advantageous for a small amount of trifluoroacetic anhydride to remain in the mixture. A slight excess of trifluoroacetic anhydride can be used to prevent hydrolysis from introduction of water (i.e. maintain anhydrous conditions) and to ensure that the reaction of trifluoroacetic acid moieties with vinyl ends is complete. In a preferred embodiment, the molar ratio of fluorinated or perfluorinated acid moieties to vinyl end moieties ranges from about at least 1:1 (acid moieties:vinyl end moieties), or about at least 5:1, or about at least 10:1, and up to about 1000:1, or up to about 10000:1, or up to about 30000:1.

The method by which excess fluorinated or perfluorinated moieties is removed can be any method known in the art. For example, excess fluorinated or perfluorinated moieties (e.g. fluorinated or perfluorinated carboxylic acid or anhydries) can be removed by evaporation or distillation. For example, when the liquid solution and/or mixture of liquid solution with polymer comprises trifluoroacetic anhydride and trifluoroacetic acid, it is possible to remove the anhydride by heating the solution thereby evaporating the anhydride preferentially since it has a substantially lower boiling point than the acid. In another example, it may be possible to remove the anhydride using a distillation column with a condenser and reflux head thereby selectively fractionating low boiling components such as trifluoroacetic anhydride from higher boiling components. Additionally, it is provided that following removal of any fluorinated or perfluorinated moieties, an amount of any compound comprising said fluorinated or perfluorinated moieties can be added back into the liquid solution or any mixture derived therefrom either directly or indirectly to form a mixture with a small percentage excess of fluorinated or perfluorinated moieties.

In a preferred embodiment of the present invention, excess amount of fluorinated or prefluorinated acid moieties can be reacted to a compound or compounds that do not interfere in the quantitative measurement of vinyl ends. For example, when using a liquid solution and/or mixture of liquid solution with polymer comprising trifluoroacetic acid and anhydride, a non-fluorinated organic acid may be added that can react with any excess anhydride. Suitable organic acids include, but are not limited to, unsubstituted or substituted, saturated, aliphatic or aromatic carboxylic acids containing a total of up to about 20 carbon atoms. The unsubstituted organic acids typically contain 2 to 18, preferably about 2 to 6, carbon atoms. The organic acid may be substituted with one or more, typically not more than one, substituent selected from alkoxy containing up to about 12 carbon atoms, halogen such as chlorine and bromine, containing up to about 12 carbon atoms. The organic acid may be substituted with a second carboxyl group, e.g., adipic acid, azelaic acid and the like. The organic acid preferably is an unsubstituted alkanoic acid containing about 2 to 6 carbon atoms (e.g. acetic acid, propanoic acid, n-butanoic acid, etc...). The organic acid may also include mixtures of suitable organic acids. For example, a mixture containing two or more alkanoic acids containing about 2 to 6 carbon atoms (e.g. a mixture containing approximately 90% acetic acid and 10% propanoic acid) may be used. The organic acid may be used amounts such that the non-fluorinated acid moieties to fluorinated moiety ratios are in the range of from about at least 2 parts non-fluorinated acid moieties to 1 part fluorinated acid moieties, or from about 3 parts non-fluorinated acid moieties to 1 part fluorinated acid moieties and up to about 8 parts non-fluorinated acid moieties to 1 part fluorinated acid moieties, or up to about 10 parts non-fluorinated acid moieties to 1 part fluorinated acid moieties

It is also provided in another embodiment of this invention, the addition or presence of at least one chemical compound that can function as a standard by which quantitative signals for fluorine or fluorine compounds are compared with. For example, in the specific instance where FMR is utilized, quantitative signals for specific fluorine compounds are often compared against quantitative signals for another chemical compound containing fluorine that is present in known amounts. The number of fluorine compounds is not limited. However, suitable fluorine compounds include any fluorinated and/or perfluorinated compound which is a liquid at ambient conditions and exhibit none or minimal chemical reactivity with other compounds in the liquid mixture. Preferably, the fluorinated and/or perfluorinated compound or compounds exhibit a signal within a FMR spectra that is distinct (i.e. does not overlap) with respect to other fluorine-containing compounds in the liquid mixture. Preferred are compounds containing fluorine such as, but not limited to, alpha,alpha,alpha-trifluorotoluene, alpha,alpha,alpha-trifluorotoluic acid, (trifluoromethoxy)benzene, (trifluoromethoxy) toluene, alpha,alpha,alpha-trifluorotolunitrile, hexafluorobenzene, octafluorotoluene, trifluoroacetonitrile, trifluoroacetamide.

Once a suitable mixture comprising the polyester polymer and at least one fluorinated carboxylic acid or fluorinated carboxylic anhydride is produced, the mixture is subjected to an analysis wherein a quantitative signal for fluorine or fluorine compounds which have reacted with the vinyl end group is produced. There are many known techniques by which a signal for fluorine is produced. Because fluorine has a nuclear spin of one-half, a strong signal, and a large coupling constant, NMR (nuclear magnetic resonance) spectroscopic analysis is a preferred. Even more preferable is FMR. Other means for providing a specific signal for fluorine analyte reacted with a vinyl end group may include, but are not limited to, gas chromatography, infrared spectroscopy, Raman spectroscopy, and mass spectrometry. In general, a commercially available instrument (e.g. a FMR instrument, an NMR instrument, a mass spectrometer, etc...) can be used to provide a quantitative signal.

Once a suitable signal is obtained, the quantity or concentration of vinyl ends in the polyester polymer is obtained by calculation. Typically, commercial instruments produce signal in the form of discrete counts or spectra. For example, an FMR instrument may produce quantitative data in the form of a spectra wherein the signal for the vinyl ends can be calculated from the area under a curve. The area signal can measured with appropriate known techniques and compared with the area of signal for known standards as part of calculation to measure the quantity and/or concentration of vinyl ends in a polyester polymer.

Once suitable calculations are completed, the numerical value or values for the concentration or amount of vinyl ends in the polyester polymer is reported or displayed. The numerical value may be reported or recorded onto a display such as a computer screen or monitor. The numerical value or a range of numerical values may be reported or recorded onto archiving media. Archiving media would be any tangible object used to record the numerical value for access at a later time. Examples of archiving media include, but are not limited to, paper notebooks, paper journals, computer magnetic storage drives, computer optical storage drives, and memory chips.

This invention can be further illustrated by the additional examples of embodiments thereof, although it will be understood that these examples are included merely for purposes of illustration and are not intended to limit the scope of the invention.

## Examples

Example 1.

The measurement of the vinyl end group in a poly(ethyleneterephthalate) polyester is completed as follows.

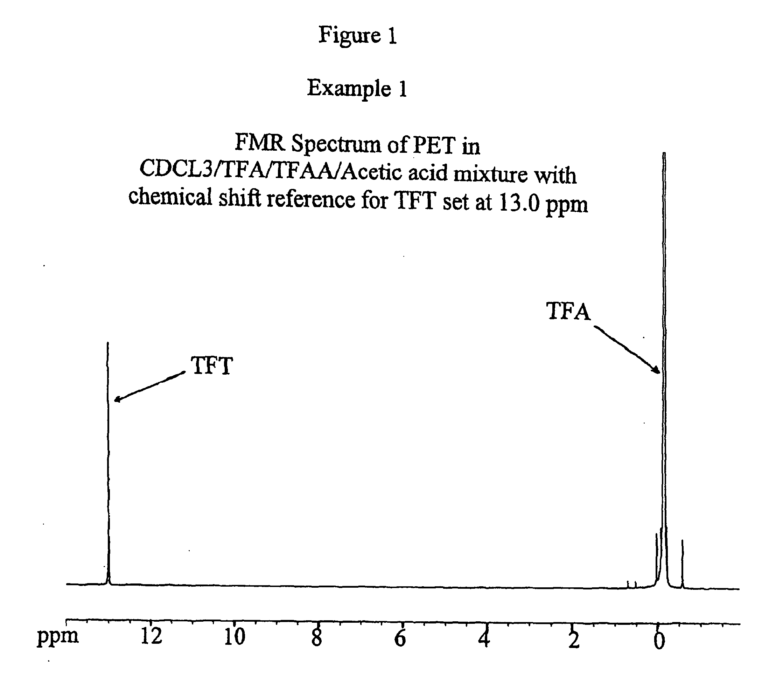

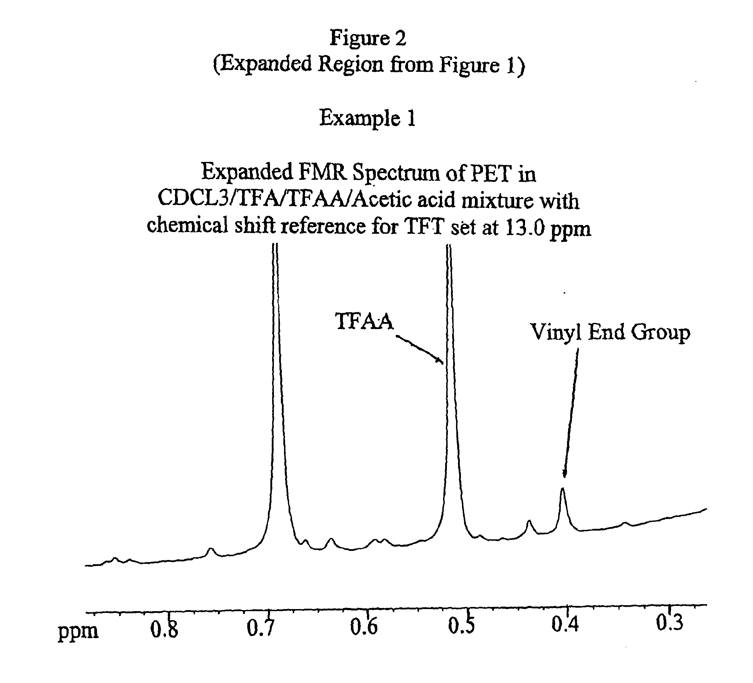

Approximately 0.4 grams of the sample is weighed to the nearest mg and placed in a 4 dram screw top vial along with a Teflon coated stir bar. A fresh solvent mixture (solution A) is prepared with exact volume ratios by measuring 75 parts Chloroform-d(CDCL3, Aldrich Chemical Company), 19 parts Trifluoroacetic acid(TFA), and 6 parts Trifluoroacetic anhydride(TFAA). Exactly 4.00 ml of the solution A is added to the sample vial and the vial is closed and sealed with a polycone screw top closure. The vial is heated to 50 °C in an aluminum heat block and stirred for 16 hrs. The vial is then removed from the heat block and cooled. A fresh solution (solution B) is prepared with exact volume ratios by mixing 2 parts of solution A and one part acetic acid. The vial closure is opened and exactly 1.00 ml of solution B and exactly 50 microliters of alpha, alpha, alpha- trifluorotoluene(TFT) is added to the vial. The vial is closed and mixed well. A portion of the prepared solution is loaded into a NMR tube, and a NMR spectrum is recorded for analysis on a Bruker Avance 500 MHz instrument using conditions which provide quantitative signals for the fluorine 19 NMR experiment or a NMR instrument with similar capability. Key NMR instrument conditions are; Pulse delay = 5 sec.; Sweep width = 32.795 ppm; Number of scans averaged = 512; Number of points = 65536; Line broading = 2.0 Hz. Representative spectra are shown in Figures 1 and 2. The chemical shift is referenced at 13.0 ppm with the alpha,alpha,alpha - trifluorotoluene peak. The area of the alpha, alpha, alpha - trifluorotoluene peak at 13 ppm including the area of the spinning side bands is measured accurately along with the accurate area of the vinyl end group peak at approximately 0.4 ppm. If the vinyl end group signal is not baseline resolved from adjacent peaks, the area may be measured using curving fitting methods or other acceptable area measurement methods. In the formula below, a correction factor multiplier of 1.1 is used to correct the area of the vinyl end group to improve accuracy. The method standard deviation is 0.43 mmol/kg at the 5.6 mmol/kg vinyl end group level and 0.11 mmol/kg at the 0.76 mmol/kg vinyl end group level.

Representative calculations are shown below;

**mmol/kg vinyl end group = ((area of vinyl end group)\*1.1\*0.0595\*1,000,000) divided by ((area of the TFT peak)\*146.1\*(sample weight in gm))**

**Comparative Example 1.**

0.06 grams of crystallized PET pellets were added to 5 ml of chloroform and stirred and heated at 50 °C for 48 hrs. The pellets did not dissolve or change shape by observation.

Preferred embodiments of the present invention are:

Item 1: A method for the measurement of vinyl ends concentration in a polyester polymer comprising:

a). dissolving said polyester polymer with a liquid solution comprising at least one fluorinated carboxylic acid or fluorinated carboxylic anhydride to form an anhydrous mixture;
b). optionally heating said liquid solution or said mixture to a temperature between about 30 °C and 300 °C;
c). subjecting said unheated or heated mixture to an analysis wherein a quantitative signal for fluorine or a fluorine-containing compound is produced; and
d). calculating the quantity or concentration of vinyl ends from said quantitative signals.

Item 2; The method of item 1, wherein said polyester polymer comprises a dicarboxylic acid moiety comprising terephthalic acid or its ester forming derivative and a diol moiety comprising ethylene glycol.
Item 3: The method of item 1, wherein said polyester polymer comprises a dicarboxylic acid comprising isophthalic acid or its ester forming derivative and a diol moiety comprising ethylene glycol.
Item 4: The method of item 1, wherein said polyester polymer comprises a dicarboxylic acid comprising naphthalene dicarboxylic acid or its ester forming derivative and a diol moiety comprising ethylene glycol.
Item 5: The method of item 1, wherein said fluorinated carboxylic acid comprises at least one of linear or branched aliphatic or aromatic carboxylic acids, wherein at least one of the carbon-bonded hydrogen atoms has been replaced by fluorine. As it is used herein, the term perfluorinated means that all of the carbon-bonded hydrogen atoms have been replaced by fluorine. Examples of suitable fluorinated acids include, but are not limited to, monofluoroacetic acid, difluoroacetic acid, trifluoroacetic acid, pentafluoropropionic acid, heptafluorobutyric acid, perfluoropentanoic acid, perfluorohexanoic acid, chlorodifluoroacetic acid, 2,2,3,3-tetrafluoropropionic acid, 3,3,3-trifluoropropionic acid, alpha,alpha,alpha-trifluorotoluic acid, pentafluorobenzoic acid, or mixtures thereof.
Item 6: The method of item 1, wherein said fluorinated carboxylic acid anhydride comprises at least one of linear or branched aliphatic or aromatic carboxylic acid anhydride or mixed anhydride, wherein at least one of the carbon-bonded hydrogen atoms has been replaced by fluorine. As it is used herein, the term perfluorinated means that all of the carbon-bonded hydrogen atoms have been replaced by fluorine. Examples of suitable fluorinated anhydrides include, but are not limited to, monofluoroacetic anhydride, difluoroacetic anhydride, trifluoroacetic anhydride, pentafluoropropionic anhydride, heptafluorobutyric anhydride, perfluoropentanoic anhydride, perfluorohexanoic anhydride, chlorodifluoroacetic anhydride, 2,2,3,3-tetrafluoropropionic anhydride, 3,3,3-trifluoropropionic anhydride, alpha,alpha,alpha-trifluorotoluic anhydride, pentafluorobenzoic anhydride, or mixtures thereof.
Item 8: The method of item 1, wherein the molar ratio or fluorinated carboxylic acid moieties to vinyl end groups in said liquid solution or said mixture is in the amount between 5:1 and 10000:1.
Item 9: The method of item 8, wherein the molar ratio or fluorinated carboxylic acid moieties to vinyl end groups in said liquid solution or said mixture is in the amount between 10:1 and 1000:1.
Item 10: The method of item 1, wherein said liquid solution or said mixture comprises at least one solvent wherein said solvent comprises at least one of butane, pentane, hexane, heptane, octane, nonane, decane, undecane, dodecane, cyclopentane, cyclohexane, cycloheptane, 2,2,4-trimethyl pentane, acetonitrile, butyl acetate, tert-butyl methyl ether, 1,4-dioxane, ethyl ether, isopropyl ether, butyl ether, 2-methoxyethyl ether, tetrahydrofuran, nitromethane, benzene, toluene, xylene, ethylbenzene, nitrobenzene, benzonitrile, anisole, pyridine, chlorobenzene, bromobenzene, dichlorobenzene, trichlorobenzene, dibromobenzene, methylene chloride, methylene chloride-d2, chloroform, chloroform-d, chlorobutane, carbon tetrachloride dichloromethane, dichloroethane, dichloroethylene, trichloroethane, trichloroethylene, tetrachloroethane, tetrachloroethane-d2, tetrachloroethylene, or chlorodifluoroacetic acid.
Item 11: The method of item 1, wherein said liquid solution or said mixture comprises at least one chemical compound standard in known quantity wherein said standard comprises at least one of alpha,alpha,alpha-trifluorotoluene, alpha,alpha,alpha-trifluorotoluic acid, (trifluoromethoxy)benzene, (trifluoromethoxy)toluene, alpha,alpha,alpha-trifluorotolunitrile, hexafluorobenzene, octafluorotoluene, trifluoroacetonitrile, trifluoroacetamide, or mixtures thereof.
Item 12: The method of item 1, wherein said quantitative signal is produced by a fluorine nuclear magnetic resonance instrument.
Item 14: The method of item 1, wherein said quantitative signal is produced by one or more gas chromatography instruments.
Item 15: The method of item 1, wherein quantitative signal is produced by one or more infrared spectroscopy instruments.

Item 16: The method of item 15, wherein said infrared spectroscopy instrument comprises a fourier transform infrared spectrometer.

Item17: The method of item 1, wherein quantitative signal is produced by one or more Raman spectroscopy instruments.

Item 19: The method of item 1, wherein the dissolving of polyester polymer is in the amount of between 0.05 grams and 10 grams of polyester polymer.

Item 20: The method of item 19, wherein the dissolving of polyester polymer is in the amount of between 0.1 grams and 5 grams of polyester polymer.

Item 21: The method of item 1 comprising the additional step of removing excess fluorinated moieties from said liquid solution or from said mixture.

Item 22: The method of claim 21, wherein said removing of excess fluorinated moieties from said liquid solution or from said mixture is by evaporation.

Item 23: The method of item 21, wherein said removing of excess fluorinated moieties from said liquid solution or from said mixture is by distillation.

Item 24: The method of item 1, comprising the additional step of reacting excess fluorinated moieties within said liquid solution or within said mixture with a non-fluorinated organic acid.

Item 25: The method of item 24, wherein said non-fluorinated organic acid comprises at least one of,unsubstituted or substituted, saturated, aliphatic or aromatic carboxylic acids containing a total of up to about 20 carbon atoms. The unsubstituted organic acids typically contain 2 to 18, preferably about 2 to 6, carbon atoms. The organic acid may be substituted with one or more, typically not more than one, substituent selected from alkoxy-containing up to about 12 carbon atoms, halogen such as chlorine and bromine, containing up to about 12 carbon atoms. Examples of suitable compounds include, but are not limited to acetic acid, propionic acid,

butyric acid, valeric acid, benzoic acid, toluic acid, or mixtures thereof. Item 26: The method of item 1, wherein said heating of said liquid solution or said mixture is to a temperature between about 50 °C and 150 °C.

Item 27: A method of describing a vinyl ends concentration or amount within a polyester polymer as a numerical value comprising reporting said numerical value in or on an archiving media or display, said numerical value having been obtained by the method of item 1.

Item 28: A method of describing a vinyl ends concentration or amount within a polyester polymer as a numerical range comprising reporting said numerical range in or on an archiving media or display, said numerical range having been obtained by the method of item 1.

Item 29: The method of item 27 or 28, wherein said archiving media comprises at least one of paper notebooks, paper journals, computer magnetic storage drives, computer optical storage drives, memory chips or a combination thereof.

Item 30: The method of item 27 or 28, wherein said display comprises a computer screen or computer monitor.

## Claims

1. A method for the measurement of vinyl ends concentration in a polyester polymer comprising:

   a) dissolving said polyester polymer with a liquid solution comprising at least one fluorinated carboxylic acid or fluorinated carboxylic anhydride to form an anhydrous mixture;
   b) optionally heating said liquid solution or said mixture to a temperature between about 30°C and 300 °C;
   c) subjecting said unheated or heated mixture to an analysis wherein a quantitative signal for fluorine or a fluorine-containing compound is produced; and
   d) calculating the quantity or concentration of vinyl ends from said quantitative signals.

2. The method of claim 1, wherein said polyester polymer comprises a dicarboxylic acid moiety comprising terephthalic acid or its ester forming derivative and a diol moiety comprising ethylene glycol.

3. The method of claim 1, wherein said polyester polymer comprises a dicarboxylic acid comprising isophthalic acid or its ester forming derivative and a diol moiety comprising ethylene glycol.

4. The method of claim 1, wherein said polyester polymer comprises a dicarboxylic acid comprising naphthalene dicarboxylic acid or its ester forming derivative and a diol moiety comprising ethylene glycol.

5. The method of claim 1, wherein said fluorinated carboxylic acid comprises monofluoroacetic acid, difluoroacetic acid, trifluoroacetic acid, pentafluoropropionic acid, heptafluorobutyric acid, perfluoropentanoic acid, perfluorohexanoic acid, chlorodifluoroacetic acid, 2,2,3,3-tetrafluoropropionic acid, 3,3,3-trifluoropropionic acid, alpha,alpha, alpha-trifluorotoluic acid, pentafluorobenzoic acid, or mixtures thereof, and wherein said fluorinated carboxylic acid anhydride comprises monofluoroacetic anhydride, difluoroacetic anhydride, trifluoroacetic anhydride, pentafluoropropionic anhydride, heptafluorobutyric anhydride, perfluoropentanoic anhydride, perfluorohexanoic anhydride, chlorodifluoroacetic anhydride, 2,2,3,3-tetrafluoropropionic anhydride. 3,3,3-trifluoropropionic anhydride, alpha,alpha,alpha-trifluorotoluic anhydride, pentafluorobenzoic anhydride, or mixtures thereof.

6. The method of claim 1, wherein the molar ratio or fluorinated carboxylic acid moieties to vinyl end groups in said liquid solution or said mixture is in the amount between 5:1 and 10000:1.

7. The method of claim 1, wherein said liquid solution or said mixture comprises at least one solvent wherein said solvent comprises at least one of butane, pentane, hexane, heptane, octane, nonane, decane, undecane, dodecane, cyclopentane, cyclohexane, cycloheptane, 2,2,4-trimethyl pentane, acetonitrile, butyl acetate, tert-butyl methyl ether, 1,4-dioxane, ethyl ether, isopropyl ether, butyl ether, 2-methoxyethyl ether, tetrahydrofuran, nitromethane, benzene, toluene, xylene, ethylbenzene, nitrobenzene, benzonitrile, anisole, pyridine, chlorobenzene, bromobenzene, dichlorobenzene, trichlorobenzene, dibromobenzene, methylene chloride, methylene chloride-d2, chloroform, chloroform-d, chlorobutane, carbon tetrachloride dichloromethane, dichloroethane, dichloroethylene, trichloroethane, trichloroethylene, tetrachloroethane, tetrachloroethane-d2, tetrachloroethylene, or chlorodifluoroacetic acid.

8. The method of claim 1, wherein said liquid solution or said mixture comprises at least one chemical compound standard wherein said standard comprises at least one of alpha,alpha,alpha-trifluorotoluene, alpha,alpha,alpha-trifluorotoluic acid, (trifluoromethoxy)benzene, (trifluoromethoxy)toluene, alpha,alpha,alpha-trifluorotolunitrile, hexafluorobenzene, octafluorotoluene, trifluoroacetonitrile, trifluoroacetamide, or mixtures thereof.

9. The method of claim 1, wherein said quantitative signal is produced by a fluorine nuclear magnetic resonance instrument.

10. The method of claim 1, wherein said quantitative signal is produced by one or more gas chromatography instruments.

11. The method of claim 1, wherein said quantitative signal is produced by an infrared spectroscopy instrument comprising a fourier transform infrared spectrometer.

12. The method of claim 1, wherein quantitative signal is produced by one or more Raman spectroscopy instruments.

13. The method of claim, wherein the dissolving of polyester polymer is in the amount of between 0.05 grams and 10 grams of polyester polymer

14. The method of claim 1 comprising the additional step of removing excess fluorinated moieties from said liquid solution or from said mixture.

15. The method of claim 14, wherein said removing of excess fluorinated moieties from said liquid solution or from said mixture is by evaporation.

16. The method of claim 15, wherein said removing of excess fluorinated moieties from said liquid solution or from said mixture is by distillation.

17. The method of claim 1, comprising the additional step of reacting excess fluorinated moieties within said liquid solution or within said mixture with a non-fluorinated organic acid.

18. The method of claim 17, wherein said non-fluorinated organic acid comprises at least one of acetic acid, propionic acid, butyric acid, valeric acid, benzoic acid, toluic acid, or mixtures thereof.

19. The method of claim 1, wherein said heating of said liquid solution or said mixture is to a temperature between about 50 °C and 150 °C.

20. A method of describing a vinyl ends concentration or amount within a polyester polymer as a numerical value comprising reporting said numerical value in or on an archiving media or display, said numerical value having been obtained by the method of claim 1.

**Patentansprüche**

1. Verfahren zur Messung der Konzentration von Vinylenden in einem Polyesterpolymer, umfassend:

    a) das Lösen des Polyesterpolymers mit einer flüssigen Lösung, umfassend mindestens eine fluorierte Carbonsäure oder fluoriertes Carbonsäureanhydrid, unter Bildung eines wasserfreien Gemisches;
    b) gegebenenfalls das Erhitzen der flüssigen Lösung oder des Gemisches auf eine Temperatur zwischen etwa 30 °C und 300 °C;
    c) das Unterziehen des nicht erhitzten oder erhitzten Gemisches einer Analyse, wobei ein quantitatives Signal für Fluor oder eine fluorhaltige Verbindung erzeugt wird; und
    d) das Berechnen der Menge oder Konzentration von Vinylenden aus den quantitativen Signalen.

2. Verfahren nach Anspruch 1, wobei das Polyesterpolymer eine Dicarbonsäurekomponente, umfassend Terephthalsäure oder ihr Ester-bildendes Derivat, und eine Diolkomponente, umfassend Ethylenglycol, umfasst.

3. Verfahren nach Anspruch 1, wobei das Polyesterpolymer eine Dicarbonsäure, umfassend Isophthalsäure oder ihr Ester-bildendes Derivat, und eine Diolkomponente, umfassend Ethylenglycol, umfasst.

4. Verfahren nach Anspruch 1, wobei das Polyesterpolymer eine Dicarbonsäure, umfassend Naphthalindicarbonsäure oder ihr Ester-bildendes Derivat, und eine Diolkomponente, umfassend Ethylenglycol, umfasst.

5. Verfahren nach Anspruch 1, wobei die fluorierte Carbonsäure Monofluoressigsäure, Difluoressigsäure, Trifluoressigsäure, Pentafluorpropionsäure, Heptafluorbutansäure, Perfluorpentansäure, Perfluorhexansäure, Chlordifluoressigsäure, 2,2,3,3-Tetrafluorpropionsäure, 3,3,3-Trifluorpropionsäure, alpha,alpha,alpha-Trifluortoluylsäure, Pentafluorbenzoesäure oder Gemische davon umfasst und wobei das fluorierte Carbonsäureanhydrid Monofluoressigsäureanhydrid, Difluoressigsäureanhydrid, Trifluoressigsäureanhydrid, Pentafluorpropionsäureanhydrid, Heptafluorbutansäureanhydrid, Perfluorpentansäureanhydrid, Perfluorhexansäureanhydrid, Chlordifluoressigsäureanhydrid, 2,2,3,3-Tetrafluorpropionsäureanhydrid, 3,3,3-Trifluorpropionsäureanhydrid, alpha,alpha,alpha-Trifluortoluylsäureanhydrid, Pentafluorbenzoesäureanhydrid oder Gemische davon umfasst.

6. Verfahren nach Anspruch 1, wobei das Molverhältnis von fluorierten Carbonsäurekomponenten zu Vinylendgruppen in der flüssigen Lösung oder dem Gemisch 5 : 1 bis 10000 : 1 beträgt.

7. Verfahren nach Anspruch 1, wobei die flüssige Lösung oder das Gemisch mindestens ein Lösungsmittel umfasst, wobei das Lösungsmittel mindestens eines von Butan, Pentan, Hexan, Heptan, Octan, Nonan, Decan, Undecan, Dodecan, Cyclopentan, Cyclohexan, Cycloheptan, 2,2,4-Trimethylpentan, Acetonitril, Butylacetat, tert-Butylmethylether, 1,4-Dioxan, Ethylether, Isopropylether, Butylether, 2-Methoxyethylether, Tetrahydrofuran, Nitromethan, Benzol, Toluol, Xylol, Ethylbenzol, Nitrobenzol, Benzonitril, Anisol, Pyridin, Chlorbenzol, Brombenzol, Dichlorbenzol, Trichlorbenzol, Dibrombenzol, Methylenchlorid, Methylenchlorid-d2, Chloroform, Chloroform-d, Chlorbutan, Kohlenstofftetrachlorid, Dichlormethan, Dichlorethan, Dichlorethylen, Trichlorethan, Trichlorethylen, Tetrachlorethan, Tetrachlorethan-d2, Tetrachlorethylen oder Chlordifluoressigsäure ist.

8. Verfahren nach Anspruch 1, wobei die flüssige Lösung oder das Gemisch mindestens einen chemischen Verbindungsstandard umfasst, wobei der Standard mindestens eines von alpha,alpha,alpha-Trifluortoluol, alpha,alpha,alpha-Trifluortoluylsäure, (Trifluormethoxy)benzol, (Trifluormethoxy)toluol, alpha,alpha,alpha-Trifluortolunitril, Hexafluorbenzol, Octafluortoluol, Trifluoracetonitril, Trifluoracetamid oder Gemische davon umfasst.

9. Verfahren nach Anspruch 1, wobei das quantitative Signal von einem Gerät für Fluor-kernmagnetische Resonanz erzeugt wird.

10. Verfahren nach Anspruch 1, wobei das quantitative Signal von einem oder mehreren Gaschromatographen erzeugt wird.

11. Verfahren nach Anspruch 1, wobei das quantitative Signal von einem Infrarotspektroskop, umfassend ein Fourier-Transformations-Infrarotspektrometer, erzeugt wird.

**12.** Verfahren nach Anspruch 1, wobei das quantitative Signal von einem oder mehreren Raman-Spektroskopen erzeugt wird.

**13.** Verfahren nach Anspruch 1, wobei das Polyesterpolymer in einer Menge zwischen 0,01 Gramm und 100 Gramm Polyesterpolymer gelöst wird.

**14.** Verfahren nach Anspruch 1, umfassend den zusätzlichen Schritt des Entfernens überschüssiger fluorierter Komponenten aus der flüssigen Lösung oder aus dem Gemisch.

**15.** Verfahren nach Anspruch 14, wobei die überschüssigen fluorierten Komponenten aus der flüssigen Lösung oder aus dem Gemisch durch Eindampfen entfernt werden.

**16.** Verfahren nach Anspruch 15, wobei die überschüssigen fluorierten Komponenten aus der flüssigen Lösung oder aus dem Gemisch durch Destillation entfernt werden.

**17.** Verfahren nach Anspruch 1, umfassend den zusätzlichen Schritt des Umsetzens überschüssiger fluorierter Komponenten in der flüssigen Lösung oder in dem Gemisch mit einer nicht fluorierten organischen Säure.

**18.** Verfahren nach Anspruch 17, wobei die nicht fluorierte organische Säure mindestens eine von Essigsäure, Propionsäure, Butansäure, Valeriansäure, Benzoesäure, Toluylsäure oder Gemische davon umfasst.

**19.** Verfahren nach Anspruch 1, wobei die flüssige Lösung oder das Gemisch auf eine Temperatur zwischen etwa 50 °C und 150 °C erhitzt wird.

**20.** Verfahren zur Darstellung einer Konzentration oder Menge von Vinylenden in einem Polyesterpolymer als Zahlenwert, umfassend das Übertragen des Zahlenwertes in oder auf ein(e) Speichermedium oder Anzeige, wobei der Zahlenwert durch das Verfahren nach Anspruch 1 erhalten worden ist.

**Revendications**

**1.** Procédé de mesure de la concentration de terminaisons vinyliques dans un polymère de polyester, comprenant les étapes consistant à :

    a) dissoudre ledit polymère de polyester avec une solution liquide comprenant au moins un acide carboxylique fluoré ou un anhydride carboxylique fluoré pour former un mélange anhydre ;
    b) éventuellement chauffer ladite solution liquide ou ledit mélange à une température entre environ 30 °C et 300 °C ;
    c) soumettre ledit mélange non chauffé ou chauffé à une analyse dans laquelle est produit un signal quantitatif pour du fluor ou un composé contenant du fluor ; et
    d) calculer la quantité ou la concentration de terminaisons vinyliques à partir desdits signaux quantitatifs.

**2.** Procédé selon la revendication 1, dans lequel ledit polymère de polyester comprend un radical acide dicarboxylique comprenant de l'acide téréphtalique ou son dérivé formant un ester et un radical diol comprenant de l'éthylèneglycol.

**3.** Procédé selon la revendication 1, dans lequel ledit polymère de polyester comprend un acide dicarboxylique comprenant de l'acide isophtalique ou son dérivé formant un ester et un radical diol comprenant de l'éthylèneglycol.

**4.** Procédé selon la revendication 1, dans lequel ledit polymère de polyester comprend un acide dicarboxylique comprenant de l'acide naphtalènedicarboxylique ou son dérivé formant un ester et un radical diol comprenant de l'éthylèneglycol.

**5.** Procédé selon la revendication 1, dans lequel ledit acide carboxylique fluoré comprend l'acide monofluoroacétique, l'acide difluoroacétique, l'acide trifluoroacétique, l'acide pentafluoropropionique, l'acide heptafluorobutyrique, l'acide perfluoropentanoïque, l'acide perfluorohexanoïque, l'acide chlorodifluoroacétique, l'acide 2,2,3,3-tétrafluoropropionique, l'acide 3,3,3-trifluoropropionique, l'acide alpha,alpha,alpha-trifluorotoluique, l'acide pentafluorobenzoïque, ou leurs mélanges, et dans lequel ledit anhydride d'acide carboxylique fluoré comprend l'anhydride monofluoroacétique, l'anhydride difluoroacétique, l'anhydride trifluoroacétique, l'anhydride pentafluoropropionique, l'anhydride heptafluorobutyrique, l'anhydride perfluoropentanoïque, l'anhydride perfluorohexanoïque, l'anhydride chlorodifluoroacétique, l'anhydride 2,2,3,3-tétrafluoropropionique, l'anhydride 3,3,3-trifluoropropionique, l'anhydride alpha,alpha, alpha-trifluorotoluique, l'anhydride pentafluorobenzoïque ou leurs mélanges.

6. Procédé selon la revendication 1, dans lequel le rapport molaire des radicaux acide carboxylique fluorés aux groupements de terminaisons vinyliques dans ladite solution liquide ou dans ledit mélange est présent en quantité de 5:1 à 10000:1.

7. Procédé selon la revendication 1, dans lequel ladite solution liquide ou ledit mélange comprend au moins un solvant, dans lequel ledit solvant comprend au moins un des composés suivantes : butane, pentane, hexane, heptane, octane, nonane, décane, undécane, dodécane, cyclopentane, cyclohexane, cycloheptane, 2,2,4-triméthylpentane, acétonitrile, acétate de butyle, méthyléther tert-butylique, 1,4-dioxanne, éther d'éthyle, éther d'isopropyle, éther de butyle, éther de 2-méthoxyéthyle, tétrahydrofurane, nitrométhane, benzène, toluène, xylène, éthylbenzène, nitrobenzène, benzonitrile, anisole, pyridine, chlorobenzène, bromobenzène, dichlorobenzène, trichlorobenzène, dibromobenzène, chlorure de méthylène, chlorure de méthylène-d2, chloroforme, chloroforme-d, chlorobutane, tétrachlorure de carbone, dichlorométhane, dichloroéthane, dichloroéthylène, trichloroéthane, trichloroéthylène, tétrachloroéthane, tétrachloroéthane-d2, tétrachloroéthylène ou acide chlorodifluoroacétique.

8. Procédé selon la revendication 1, dans lequel ladite solution liquide ou ledit mélange comprend au moins un composé chimique standard, dans lequel ledit standard comprend au moins un des composés suivants : alpha,alpha-trifluorotoluène, acide alpha,alpha,alpha-trifluorotoluique, (trifluorométhoxy)benzène, (trifluorométhoxy)toluène, alpha, alpha,alpha-trifluorotolunitrile, hexafluorobenzène, octafluorotoluène, trifluoroacétonitrile, trifluoroacétamide ou leurs mélanges.

9. Procédé selon la revendication 1, dans lequel ledit signal quantitatif est produit par un instrument de résonance magnétique nucléaire au fluor.

10. Procédé selon la revendication 1, dans lequel ledit signal quantitatif est produit par un ou plusieurs instruments de chromatographie en phase gazeuse.

11. Procédé selon la revendication 1, dans lequel ledit signal quantitatif est produit par un instrument de spectroscopie infrarouge comprenant un spectromètre infrarouge à transformée de Fourier.

12. Procédé selon la revendication 1, dans lequel ledit signal quantitatif est produit par un ou plusieurs instruments de spectroscopie de Raman.

13. Procédé selon la revendication 1, dans lequel la dissolution du polymère de polyester se situe en quantité de 0,05 gramme à 10 grammes de polymère de polyester.

14. Procédé selon la revendication 1, comprenant l'étape additionnelle d'élimination des radicaux fluorés en excès de ladite solution liquide ou dudit mélange.

15. Procédé selon la revendication 14, dans lequel ladite élimination desdits radicaux fluorés en excès de ladite solution liquide ou dudit mélange est effectuée par évaporation.

16. Procédé selon la revendication 15, dans lequel ladite élimination desdits radicaux fluorés en excès de ladite solution liquide ou dudit mélange est effectuée par distillation.

17. Procédé selon la revendication 1, comprenant l'étape additionnelle de réaction des radicaux fluorés en excès dans ladite solution liquide ou dans ledit mélange avec un acide organique non fluoré.

18. Procédé selon la revendication 17, dans lequel ledit acide organique non fluoré comprend au moins l'un des acides suivants : l'acide acétique, l'acide propionique, l'acide butyrique, l'acide valérique, l'acide benzoïque, l'acide toluique ou leurs mélanges.

19. Procédé selon la revendication 1, dans lequel ledit chauffage de ladite solution liquide ou dudit mélange se fait à une température d'environ 50 °C à 150 °C.

20. Procédé de description d'une concentration ou d'une quantité de terminaisons vinyliques dans un polymère de polyester sous la forme d'une valeur numérique comprenant le report de ladite valeur numérique dans ou sur un support d'archivage ou un affichage, ladite valeur numérique ayant été obtenue par le procédé de la revendication 1.

Figure 1

Example 1

FMR Spectrum of PET in
CDCL3/TFA/TFAA/Acetic acid mixture with
chemical shift reference for TFT set at 13.0 ppm

TFA

TFT

ppm    12      10      8      6      4      2      0

EP 2 125 931 B1

## Figure 2
### (Expanded Region from Figure 1)

### Example 1

Expanded FMR Spectrum of PET in
CDCL3/TFA/TFAA/Acetic acid mixture with
chemical shift reference for TFT set at 13.0 ppm

TFAA

Vinyl End Group

ppm    0.8    0.7    0.6    0.5    0.4    0.3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5852164 A

**Non-patent literature cited in the description**

- *J. Polymer Science A,* vol. 39 (5), 665-674
- **J. G. M. Aalbers ; G. D. B. van Houwelingen.** *Fresenius Z Anal. Chem.,* 1983, vol. 314 (5), 472-475